# EUROPÄISCHE PATENTANMELDUNG

(11) **EP 3 061 490 A1**
(43) Veröffentlichungstag der Anmeldung: **31.08.2016**
(21) Anmeldenummer: 16000323.2
(22) Anmeldetag: 10.02.2016
(51) Int. Cl.: A61N 1/04, A61N 1/36, A61N 1/08

(54) **VORRICHTUNG ZUR AUFBRINGUNG EINES TRANSKUTANEN ELEKTRISCHEN STIMULATIONSREIZES**

(30) Priorität: 27.02.2015 DE 102015002589
(71) Anmelder: cerboMed GmbH, 91052 Erlangen (DE)
(72) Erfinder: Zschaeck, Thomas, 90427 Nürnberg (DE); Hartlep, Andreas, 83607 Holzkirchen (DE)
(74) Vertreter: Gosdin, Michael

(57) **Zusammenfassung**

Die Erfindung betrifft eine Vorrichtung (1) zur Aufbringung eines transkutanen elektrischen Stimulationsreizes auf die Oberfläche eines Abschnittes des menschlichen Ohrs (2), die mindestens zwei Elektroden (3, 4) aufweist, wobei die Vorrichtung (1) eine Steuerungseinrichtung (5) umfasst, die zur Einleitung eines über die Elektroden (3, 4) fließenden Stimulationsstroms (I) ausgebildet ist. Um eine automatische optimale Anpassung der transkutanen Stimulation an einen individuellen Patienten vornehmen zu können, sieht die Erfindung vor, dass die Stimulationseinrichtung (1) umfasst: ein Speicherelement (6), in dem ein zulässiger Wertebereich (WB) für den Kontaktwiderstand (R_{K}) gespeichert werden kann, der zwischen den mindestens zwei Elektroden (3, 4) herrscht, wenn die Vorrichtung (1) bestimmungsgemäß verwendet wird; Messmittel (7) zur Messung des Kontaktwiderstands (R_{K}), der zwischen den mindestens zwei Elektroden (3, 4) herrscht, wenn die Vorrichtung (1) bestimmungsgemäß verwendet wird; Mittel (8) zum Verändern des im Speicherelement (6) gespeicherten Wertebereichs (WB) für den Kontaktwiderstand (R_{K}), wobei die Mittel (8) den gespeicherten Wertebereich (WB) ändern können, wenn ein vom Messmittel (7) gemessener Kontaktwiderstand (R_{K}) außerhalb des gespeicherten zulässigen Wertebereichs liegt.

## Beschreibung

Die Erfindung betrifft eine Vorrichtung zur Aufbringung eines transkutanen elektrischen Stimulationsreizes auf die Oberfläche eines Abschnittes des menschlichen Ohrs, die mindestens zwei Elektroden aufweist, wobei die Vorrichtung eine Steuerungseinrichtung umfasst, die zur Einleitung eines über die Elektroden fließenden Stimulationsstroms ausgebildet ist.

Es ist generell bekannt, durch invasive und non-invasive Reizung der Nerven Einfluss auf deren neurophysiologische und neuroelektrische Qualität und damit auf die Funktion der stimulierten Nerven zu nehmen. Hierdurch können verschiedene Krankheitszustände behandelt werden. Es existieren zahlreiche Vorrichtungen sowohl zur invasiven als auch zur non-invasiven Stimulation.

Die vorliegende Erfindung stellt auf die Methode der transkutanen elektrischen Nervenstimulation ab. Bei diesem Verfahren werden Impulsströme verschiedener Stromformen, Amplituden, Impulsdauern und Frequenzen durch die Haut hindurch an verschiedenen Nerven appliziert und verändern deren Statusparameter in vorteilhafter Weise.

Eine Vorrichtung der eingangs genannten Art ist aus der DE 10 2010 054 165 B3 bekannt. Hier ist eine Vorrichtung zur transkutanen Stimulation des Vagusnervs des menschlichen Körpers beschrieben, deren Elektrodenkopf mit zwei Elektroden im Bereich der Cymba conchae angeordnet wird; eine solche Positionierung der Elektroden hat sich als vorteilhaft erwiesen. Der Bereich der Cymba conchae ist dabei der Bereich der Concha des Ohres, der oberhalb des Crus helicis liegt; er wird auch als Hemiconcha superior bezeichnet. Unterhalb des Crus helicis nach unten erstreckt sich dann der Bereich des Cavum conchae.

Für einen zufriedenstellenden Behandlungserfolg durch Applikation eines transkutan wirkenden Stimulationsstroms ist es erforderlich, eine hinreichende Stromstärke zum Einsatz zu bringen, die natürlich andererseits auch nicht zu groß sein darf. Problematisch ist es in diesem Zusammenhang, dass der individuell vorliegende Kontaktwiderstand zwischen den Elektroden, die diese bei bestimmungsgemäßer Verwendung der Vorrichtung aufweisen, patientenspezifisch und auch abhängig von weiteren Umgebungsparametern ist.

Der Hautwiderstand bei verschiedenen Menschen ist nämlich verschieden und kann durchaus um den Faktor 10 variieren. Eine weitere Einflussgröße ist der genaue Auflageort der Elektroden. Wenn eine Elektrode beispielsweise direkt auf einer Schweißdrüse aufliegt, tendiert der Hautwiderstand zu niedrigen Werten; noch geringer wird der Hautwiderstand, wenn der Patient schwitzt (das heißt beispielsweise im Sommer und bei Sonne). Wieder andere Werte für den Hautwiderstand ergeben sich bei salziger Haut im Ohr (z. B. durch getrocknetes Meerwasser).

Demgemäß ist eine jeweilige spezifische Anpassung des Stimulationsstroms bei der transkutanen Stimulation aufwendig und schwierig.

Eine gattungsgemäße Stimulationsvorrichtung ist auch aus der DE 10 2011 018 228 A1 bekannt. Eine ähnliche Lösung zeigt die US 2009/0082831 A1.

Der vorliegenden Erfindung liegt daher die **Aufgabe** zugrunde, eine Vorrichtung der eingangs genannten Art bereitzustellen, mit der es möglich wird, sowohl patientenspezifisch als auch in Abhängigkeit von den Umgebungsbedingungen die jeweils optimalen Parameter für eine transkutanen Stimulation bereitzustellen. Demgemäß soll eine automatische optimale Anpassung der transkutanen Stimulation an einen individuellen Patienten vorgenommen werden können.

Die **Lösung** dieser Aufgabe durch die Erfindung ist dadurch gekennzeichnet, dass die Stimulationseinrichtung umfasst:
- ein Speicherelement, in dem ein zulässiger Wertebereich für den Kontaktwiderstand gespeichert werden kann, der zwischen den mindestens zwei Elektroden herrscht, wenn die Vorrichtung bestimmungsgemäß verwendet wird;
- Messmittel zur Messung des Kontaktwiderstands, der zwischen den mindestens zwei Elektroden herrscht, wenn die Vorrichtung bestimmungsgemäß verwendet wird;
- Mittel zum Verändern des im Speicherelement gespeicherten Wertebereichs für den Kontaktwiderstand, wobei die Mittel den gespeicherten Wertebereich ändern können, wenn ein vom Messmittel gemessener Kontaktwiderstand außerhalb des gespeicherten zulässigen Wertebereichs liegt.

Die genannten Mittel zum Verändern des im Speicherelement gespeicherten Wertebereichs für den Kontaktwiderstand sind insbesondere ausgebildet, den gespeicherten Wertebereich erst nach einer Benutzerabfrage zu ändern, wenn - wie gesagt - ein vom Messmittel gemessener Kontaktwiderstand außerhalb des gespeicherten zulässigen Wertebereichs liegt.

Die Mittel zum Verändern des im Speicherelement gespeicherten Wertebereichs für den Kontaktwiderstand können dabei ein Freigabe-Element umfassen, das vom Benutzer der Vorrichtung betätigbar ist, wobei die Mittel ausgebildet sind, eine Veränderung des gespeicherten Wertebereichs erst vorzunehmen, wenn das Freigabe-Element betätigt wurde.

Die Vorrichtung umfasst vorzugsweise weiterhin Mittel zur Ausgabe eines Signals. Die Vorrichtung kann dabei ausgebildet sein, diese Mittel zur Ausgabe eines Signals zu aktivieren, nachdem ein Vergleich eines vom Messmittel gemessenen Kontaktwiderstands mit dem im Speicherelement gespeicherten Wertebereich für den Kontaktwiderstand erfolgte. Weiterhin kann vorgesehen sein, dass die Vorrichtung ausgebildet ist, über die Mittel zur Ausgabe eines Signals ein Warnsignal abzugeben, wenn ein vom Messmittel gemessener Kontaktwiderstand außerhalb des im Speicherelement gespeicherten Wertebereichs für den Kontaktwiderstand liegt. Die Vorrichtung kann weiterhin ausgebildet sein, über die Mittel zur Ausgabe eines Signals ein Freigabesignal abzugeben, wenn ein vom Messmittel gemessenen Kontaktwiderstand innerhalb des im Speicherelement gespeicherten Wertebereichs für den Kontaktwiderstand liegt. Die Mittel zur Ausgabe eines Signals sind dabei bevorzugt ein Display oder ein Bildschirm. Alternativ oder additiv ist auch ein akustisches Ausgabemittel möglich.

Die Vorrichtung kann weiterhin ausgebildet sein, zur Bereitstellung eines erstmaligen Wertebereichs für den Kontaktwiderstand über die Messmittel zur Messung des Kontaktwiderstands Werte zu erfassen und diese im Speicherelement abzuspeichern, wobei der Wertebereich definiert wird, indem ein Überschreitungswert für die Überschreitung des gespeicherten Maximalwerts und ein Unterschreitungswert für die Unterschreitung des gespeicherten Minimalwerts vorgegeben wird.

Die Vorrichtung kann ferner ausgebildet sein, zur Bereitstellung eines jeweils aktuellen, individuellen Wertebereichs für den Kontaktwiderstand über die Messmittel zur Messung des Kontaktwiderstands Werte zu erfassen und diese im Speicherelement abzuspeichern, wobei der Wertebereich definiert wird, indem ein Überschreitungswert für die Überschreitung des gespeicherten Maximalwerts und ein Unterschreitungswert für die Unterschreitung des gespeicherten Minimalwerts vorgegeben wird, wobei insbesondere eine vorgegebene Anzahl der zuletzt gemessenen Werte zugrundegelegt wird.

Der Überschreitungswert für die Überschreitung beträgt dabei bevorzugt 20 % des gespeicherten Maximalwerts; der Unterschreitungswert für die Unterschreitung beträgt bevorzugt 20 % des gespeicherten Minimalwerts.

Die Ausgestaltung der vorgeschlagenen Vorrichtung stellt also darauf ab, dass sowohl bei einem zu hohen Widerstand eine Meldung an den Patienten abgeben wird (wie z. B. "Sitz der Ohrelektrode prüfen") als auch bei einem zu niedrigen Widerstand (z. B. "Kurzschluss" - "Kurzschluss" bedeutet hier nicht Kurzschluss im elektrotechnischen Sinne sondern, dass der Widerstand zwischen den Elektroden ungewöhnlich niedrig ist).

Wird über die Messmittel ein Kontaktwiderstand ermittelt, der deutlich vom gespeicherten zulässigen Wertebereich abweicht, muss der Patient - über das Freigabe-Element - bestätigen, dass er richtig stimuliert (er muss durch die transkutane Stimulation ein "Kribbeln" spüren). Die Vorrichtung speichert dann diesen neuen Wert im Speicherelement als richtig und berücksichtigt bei den nächsten Behandlungen den geänderten zulässigen Wertebereich.

Beispielsweise kann aus 8 Werten (aus Stimulationen von 2 Tagen) der niedrigste Wert abzüglich eines Unterschreitungswerts von 20 % als Minimum des zulässigen Wertebereichs vorgesehen werden, während der höchste Wert zuzüglich eines Überschreitungswerts von 20 % als Maximum festgelegt wird.

Unterschreitet der Patient bei einer Anwendung das Minimum, muss er die korrekte Stimulation bestätigen. Das Analoge gilt für die Überschreitung des Maximums. Der älteste dieser 8 Werte wird mit jeder abgeschlossenen Stimulationsperiode fortlaufend erneuert. Gegebenenfalls können natürlich auch mehr als 8 zuletzt vorliegende Werte berücksichtigt werden, beispielsweise 12 oder 16 Werte (d. h. Auswertung über 3 oder 4 Tage).

Dabei kann gegebenenfalls auch eine Wichtung der gemessenen Werte für den Kontaktwiderstand unter Berücksichtigung der jeweiligen Dauer der Stimulation erfolgen. Damit lässt sich erreichen, dass man bei sehr kurzen Stimulations-Sitzungen seinen Widerstand nicht nur auf Momentanwerte einstellt.

Mit der vorgeschlagenen Lösung wird es also möglich, Fehler wie Kurzschluss der Ohrelektroden oder einen zu hohen Kontaktwiderstand in einfacherer Weise erkennen zu können. Durch die vorgeschlagene Ausgestaltung wird die Stimulationsvorrichtung in den Stand gesetzt, im Zuge einiger Stimulationen-Sitzungen den typischen patientenspezifischen Hautwiderstand zu erfassen und dann als patiententypische Größe bzw. patientenspezifischen Wertebereich abzuspeichern. Bei einer deutlichen Abweichung von diesem Wert kann dann eine Warnung (z. B. "Bestätigen Sie bitte, dass die Elektrode richtig sitzt") bzw. eine Fehlermeldung ausgegeben werden.

In der Zeichnung ist ein Ausführungsbeispiel der Erfindung dargestellt. Es zeigen:
- Fig. 1: schematisch den Aufbau einer Stimulationsvorrichtung für die transkutane Stimulation eines Abschnitts des menschlichen Ohrs und
- Fig. 2: schematisch eine Anzahl von gemessenen Werten für den Kontaktwiderstand zwischen zwei Elektroden der Vorrichtung über den zeitlichen Verlauf.

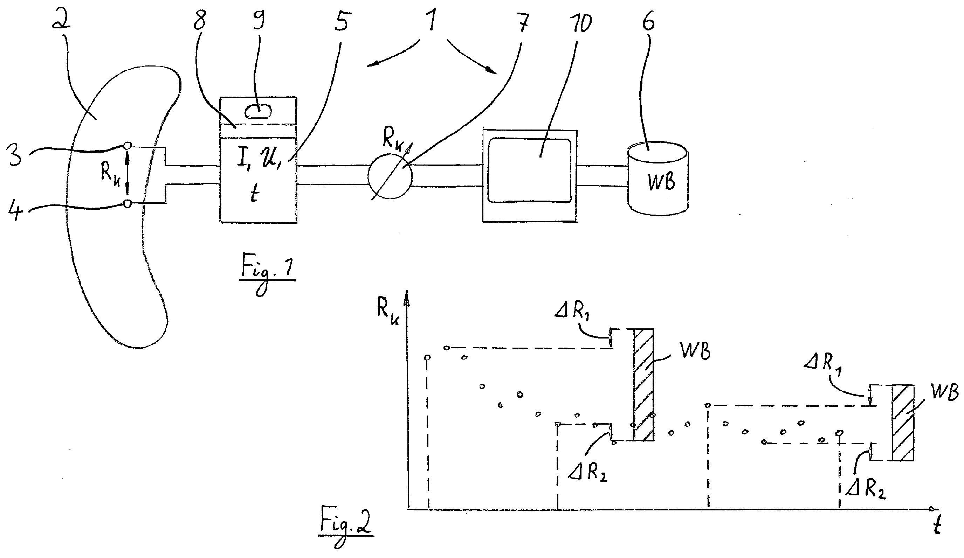

In Fig. 1 ist angedeutet, wie eine Vorrichtung 1 zur Aufbringung eines Stimulationsreizes aufgebaut ist bzw. welche Komponenten diese umfasst.
Um auf einen Abschnitt des menschlichen Ohrs 2 einen transkutanen Stimulationsreiz auszuüben, sind zwei Elektroden 3 und 4 vorgesehen, die in Hautkontakt mit dem Ohr 2 gebracht werden. Eine solche Stimulationsvorrichtung ist bekannt, wobei ausdrücklich auf die DE 10 2010 054 165 B3 der Patentinhaberin Bezug genommen wird, wo sich nähere Erläuterungen hierzu finden. Die Vorrichtung ist demnach ausgebildet, um im Bereich des Vagusnervs am Ohr der die Vorrichtung benutzenden Person angebracht zu werden. Damit kann eine transkutane Stimulation des Vagusnervs vorgenommen werden.

Eine Steuerungseinrichtung 5 veranlasst die Beaufschlagung der Elektroden 3, 4 mit einem Stimulationsstrom I bei gegebener Spannung U gemäß einem vorgegebenen zeitlichen Profil. Wesentlich ist, dass mit den Elektroden 3, 4 und der Steuerungseinrichtung 5 noch weitere Komponenten in Kommunikation stehen, nämlich ein Speicherelement 6 zum Speichern eines Wertebereichs WB für zulässige Kontaktwiderstände R_{K}, ein Messmittel 7 zur Messung des Kontaktwiderstands R_{K} und ein Mittel 10 zur Ausgabe eines Signals, vorliegend in Form eines Displays. Ferner steht ein Mittel 8 zum Verändern des gespeicherten Wertebereichs WB mit der Anordnung in Kommunikation, wobei an diesen Mitteln 8 auch ein Freigabe-Element 9 angeordnet ist.

In Fig. 2 ist schematisch dargestellt, wie sich ein zulässiger Wertebereich WB über der Zeit t in Abhängigkeit einer Anzahl gemessener Werte für den Kontaktwiderstand R_{K} ergeben kann. Hierzu ist zu sagen, dass die einzelnen Messpunkte Kontaktwiderstände R_{K} darstellen, die bei individuellen Sitzungen vom Messmittel 7 ermittelt wurden, bei denen eine transkutane Stimulation vorgenommen wurde. In der linken Bildhälfte von Fig. 2 ist zu sehen, dass die Kontaktwiderstände R_{K} tendenziell auf einem relativ hohen Niveau liegen, woraus sich der links in Figur 2 dargestellte zulässige Wertebereich WB ergibt. Hierzu wurden die letzten 8 Messwerte betrachtet und das jeweilige Maximum und Minimum ermittelt. Der zulässige Wertebereich WB ergibt sich dadurch, dass zum größten Wert des Kontaktwiderstands R_{K} ein Überschreitungswert ΔR₁ hinzugenommen wurde, der vorliegend 20 % des ermittelten Maximalwerts beträgt; entsprechend wurde vom kleinsten Wert des Kontaktwiderstands R_{K} ein Unterschreitungswert ΔR₂ abgezogen, der gleichermaßen 20 % des ermittelten Minimalwerts beträgt.

Wie zu erkennen ist, hat sich im Laufe der Zeit der Kontaktwiderstand R_{K} tendenziell verringert, wie sich aus der rechten Hälfte der Fig. 2 ergibt. Dies kann beispielsweise dadurch geschehen sein, dass sich die Witterungsverhältnisse geändert haben und infolge einer höheren Temperatur und erfolgter Schweißbildung nunmehr geringere Kontaktwiderstände vorliegen. Der sich nunmehr ergebende Wertebereich WB ist in derselben Weise wie vorstehend beschrieben ermittelt worden (siehe die markierten Überschreitungswerte bzw. Unterschreitungswerte ΔR₁/ ΔR₂).

Im Speicherelement 6 ist der jeweils aktuelle zulässige Wertebereich WB für den Kontaktwiderstand R_{K} gespeichert. Wird die Vorrichtung 1 zur transkutanen Stimulation bestimmungsgemäß verwendet und misst das Messmittel 7 einen Wert für den Kontaktwiderstand R_{K}, der innerhalb des Wertebereichs WB liegt, erfolgt die Stimulation ohne weiteres und namentlich ohne Ausgabe eines Hinweises an den Benutzer. Freilich kann auch ein Hinweis dahingehend ausgegeben werden, dass die transkutane Stimulation in ordnungsgemäßer Weise erfolgt.

Wird indes nach Einsetzen der Vorrichtung ans bzw. ins Ohr 2 über die Messmittel 7 ein Kontaktwiderstand R_{K} detektiert, der außerhalb des gespeicherten aktuellen Wertebereichs WB liegt, erfolgt über das Display 10 ein entsprechender Hinweis an den Benutzer. Abhängig davon, ob der gemessene Kontaktwiderstand R_{K} zu hoch oder zu niedrig ist - jeweils verglichen mit dem gespeicherten Wertebereich WB -, erfolgt der entsprechende Hinweis.

Ob und in welcher Weise ein Hinweis über das Display 10 erfolgt, hängt also davon ab, wie der Vergleich eines aktuell gemessenen Kontaktwiderstands R_{K} mit dem aktuell gespeicherten Wertebereich WB ausgefallen ist.

Bei einem zu hohen Kontaktwiderstand R_{K}, der in der Folge einen höheren Stimulationsstrom I erfordert, kann der Hinweis lauten: "Kontaktwiderstand zu hoch, soll die Stimulation erfolgen?". Wird dies vom Benutzer bejaht, was über das Freigabe-Element 9 der Vorrichtung 1 signalisiert werden kann, erfolgt die transkutane Stimulation, wobei der nunmehr verwendete Wert des Kontaktwiderstands R_{K} in die Bestimmung des zulässigen Wertebereichs WB einbezogen wird.

Bei einem zu geringen Kontaktwiderstand R_{K} kann der Hinweis lauten: "Kontaktwiderstand zu gering, ist die Vorrichtung richtig ins Ohr eingesetzt?". In diesem Falle läge nämlich ein "Kurzschluss" vor, was den Patienten darauf hinweisen würde, dass er beispielsweise eine Säuberung des zu stimulierenden Bereichs vornehmen sollte, bevor er die Behandlung fortsetzt.

Das Management für die Speicherung des jeweils zulässigen Wertebereichs WB für den Kontaktwiderstand R_{K} veranlassen jeweils die Mittel 8, mit denen der Wertebereich WB verändert werden kann, wenn sich dies aufgrund der oben genannten Vorgehensweise ergibt.

Wenn indes vom Benutzer der Vorrichtung bei einem entsprechenden Hinweis auf dem Display keine Berücksichtigung des neuerlichen Werts für den Kontaktwiderstand R_{K} erfolgen soll, betätigt er nicht das Freigabe-Element 9, so dass der zulässige Wertebereich WB nicht verändert wird.

Vor der erstmaligen Benutzung der Stimulationsvorrichtung kann auch werksseitig ein typischer zulässiger Wertebereich WB für den Kontaktwiderstand R_{K} im Speicherelement 6 hinterlegt werden. Der Wertebereich WB kann dann - wie oben beschrieben - Patienten-individuell verändert werden, was durch die beschriebene Vorgehensweise weitgehend automatisch erfolgt, d.h. ohne Eingriff des Arztes oder des Patienten.

### Bezugszeichenliste:

- 1: Vorrichtung zur Aufbringung eines Stimulationsreizes
- 2: Ohr
- 3: Elektrode
- 4: Elektrode
- 5: Steuerungseinrichtung
- 6: Speicherelement
- 7: Messmittel
- 8: Mittel zum Verändern des gespeicherten Wertebereichs
- 9: Freigabe-Element
- 10: Mittel zur Ausgabe eines Signals (Display)

- I: Stimulationsstrom
- U: Spannung
- R_{K}: Kontaktwiderstand
- WB: Wertebereich
- ΔR₁: Überschreitungswert
- ΔR₂: Unterschreitungswert
- t: Zeit

## Patentansprüche

1. Vorrichtung (1) zur Aufbringung eines transkutanen elektrischen Stimulationsreizes auf die Oberfläche eines Abschnittes des menschlichen Ohrs (2), die mindestens zwei Elektroden (3, 4) aufweist, wobei die Vorrichtung (1) eine Steuerungseinrichtung (5) umfasst, die zur Einleitung eines über die Elektroden (3, 4) fließenden Stimulationsstroms (I) ausgebildet ist,
**dadurch gekennzeichnet,**
**dass** die Stimulationseinrichtung (1) umfasst:
- ein Speicherelement (6), in dem ein zulässiger Wertebereich (WB) für den Kontaktwiderstand (R_{K}) gespeichert werden kann, der zwischen den mindestens zwei Elektroden (3, 4) herrscht, wenn die Vorrichtung (1) bestimmungsgemäß verwendet wird;
- Messmittel (7) zur Messung des Kontaktwiderstands (R_{K}), der zwischen den mindestens zwei Elektroden (3, 4) herrscht, wenn die Vorrichtung (1) bestimmungsgemäß verwendet wird;
- Mittel (8) zum Verändern des im Speicherelement (6) gespeicherten Wertebereichs (WB) für den Kontaktwiderstand (R_{K}), wobei die Mittel (8) den gespeicherten Wertebereich (WB) ändern können, wenn ein vom Messmittel (7) gemessener Kontaktwiderstand (R_{K}) außerhalb des gespeicherten zulässigen Wertebereichs liegt.

2. Vorrichtung nach Anspruch 1, **dadurch gekennzeichnet, dass** die Mittel (8) zum Verändern des im Speicherelement (6) gespeicherten Wertebereichs (WB) für den Kontaktwiderstand (R_{K}) ein Freigabe-Element (9) umfassen, das vom Benutzer der Vorrichtung (1) betätigbar ist, wobei die Mittel (8) ausgebildet sind, eine Veränderung des gespeicherten Wertebereichs (WB) erst vorzunehmen, wenn das Freigabe-Element (9) betätigt wurde.

3. Vorrichtung nach Anspruch 1 oder 2, **dadurch gekennzeichnet, dass** die Vorrichtung (1) weiterhin Mittel (10) zur Ausgabe eines Signals umfasst.

4. Vorrichtung nach Anspruch 3, **dadurch gekennzeichnet, dass** die Vorrichtung (1) ausgebildet ist, die Mittel (10) zur Ausgabe eines Signals zu aktivieren, nachdem ein Vergleich eines vom Messmittel (7) gemessenen Kontaktwiderstands (R_{K}) mit dem im Speicherelement (6) gespeicherten Wertebereich (WB) für den Kontaktwiderstand (R_{K}) erfolgte.

5. Vorrichtung nach Anspruch 4, **dadurch gekennzeichnet, dass** die Vorrichtung (1) ausgebildet ist, über die Mittel (10) zur Ausgabe eines Signals ein Warnsignal abzugeben, wenn ein vom Messmittel (7) gemessener Kontaktwiderstand (R_{K}) außerhalb des im Speicherelement (6) gespeicherten Wertebereichs (WB) für den Kontaktwiderstand (R_{K}) liegt.

6. Vorrichtung nach Anspruch 4, **dadurch gekennzeichnet, dass** die Vorrichtung (1) ausgebildet ist, über die Mittel (10) zur Ausgabe eines Signals ein Freigabesignal abzugeben, wenn ein vom Messmittel (7) gemessenen Kontaktwiderstand (R_{K}) innerhalb des im Speicherelement (6) gespeicherten Wertebereichs (WB) für den Kontaktwiderstand (R_{K}) liegt.

7. Vorrichtung nach einem der Ansprüche 3 bis 6, **dadurch gekennzeichnet, dass** die Mittel (10) zur Ausgabe eines Signals ein Display oder Bildschirm sind.

8. Vorrichtung nach einem der Ansprüche 1 bis 7, **dadurch gekennzeichnet, dass** die Vorrichtung (1) ausgebildet ist, zur Bereitstellung eines erstmaligen Wertebereichs (WB) für den Kontaktwiderstand (R_{K}) über die Messmittel (7) zur Messung des Kontaktwiderstands (R_{K}) Werte zu erfassen und diese im Speicherelement (6) abzuspeichern, wobei der Wertebereich (WB) definiert wird, indem ein Überschreitungswert (ΔR₁) für die Überschreitung des gespeicherten Maximalwerts und ein Unterschreitungswert (ΔR₂) für die Unterschreitung des gespeicherten Minimalwerts vorgegeben wird.

9. Vorrichtung nach einem der Ansprüche 1 bis 8, **dadurch gekennzeichnet, dass** die Vorrichtung (1) ausgebildet ist, zur Bereitstellung eines jeweils aktuellen, individuellen Wertebereichs (WB) für den Kontaktwiderstand (R_{K}) über die Messmittel (7) zur Messung des Kontaktwiderstands (R_{K}) Werte zu erfassen und diese im Speicherelement (6) abzuspeichern, wobei der Wertebereich (WB) definiert wird, indem ein Überschreitungswert (ΔR₁) für die Überschreitung des gespeicherten Maximalwerts und ein Unterschreitungswert (ΔR₂) für die Unterschreitung des gespeicherten Minimalwerts vorgegeben wird, wobei insbesondere eine vorgegebene Anzahl der zuletzt gemessenen Werte zugrundegelegt wird.

10. Vorrichtung nach Anspruch 8 oder 9, **dadurch gekennzeichnet, dass** der Überschreitungswert (ΔR₁) für die Überschreitung 20 % des gespeicherten Maximalwerts beträgt und dass der Unterschreitungswert (ΔR₂) für die Unterschreitung 20 % des gespeicherten Minimalwerts beträgt.
